# EUROPEAN PATENT SPECIFICATION

(11) **EP 0 507 469 B1**
(45) Date of publication and mention of the grant of the patent: **28.12.1994**
(21) Application number: 92302351.9
(22) Date of filing: 18.03.1992
(51) Int. Cl.: A61K 7/043

(54) **Primer for nail lacquers and method**
Grundierung für Nagellacke sowie dessen Verfahren
Composition de couche primaire pour vernis à ongles et son méthode

(30) Priority: 04.04.1991 US 680481
(43) Date of publication of application: 07.10.1992
(73) Proprietor: DOW CORNING CORPORATION, Midland Michigan 48686-0994 (US)
(72) Inventor: Legrow, Gary Edward, Midland, Michigan (US); Pape, Peter Gerald, Sanford, Michigan (US); Daunheimer, Scott Allen, Midland, Michigan (US)
(74) Representative: Dowden, Marina

(56) References cited:
- EP-A- 0 156 968
- EP-A- 0 199 325
- EP-A- 0 255 227
- FR-A- 2 448 552

## Description

This invention relates to a primer for nail lacquers and nail enamel formulations and more particularly is directed to a method of improving the adhesion of nail lacquers and enamels to human fingernails and toenails.

A nail lacquer or enamel in order to be successfully marketed must include a primary film former whose characteristics allow for ease of brushing application, fast drying and hardening and which provides for enhanced adhesion to the nail surface without a loss of resistance to chipping and abrasion. Other desirable qualities are an attractive gloss and lustre and an aesthetic color. It should be resistant to frequent immersion in water and a fair degree of abrasion. Enamels of good wear properties are comparatively free from the tendency to peel and chip. Lacquers and enamels must also be dermatologically innocuous. Perhaps however the foremost and most sought-after characteristic is enhanced adhesion.

Silane coupling agents are well-known in the art as promoters of adhesion as evidenced for example by US Patent No. 4,689,085 issued August 25, 1987. Typically, these materials are employed as an interface between hydrophilic mineral surfaces such as glass and silica and organic resins in order to couple these dissimilar surfaces.

Silane coupling agents are of the general structure X₃Si(CH₂)ₙY in which n is an integer from zero to three, X is a hydrolyzable group on silicon and Y is a reactive organofunctional group. When applied from water solutions, the hydrolyzable groups generate intermediate silanols which migrate to the hydrophilic surface where they condense with surface hydroxyl groups to form "siloxane" bonds (-SiOSi-) with the surface. It is believed that coupling is achieved with the organic resin because of the compatibility between the resin and the reactive organofunctional group Y of the silane coupling agent.

Examples of some of the well-known and commercial varieties of silane coupling agents are N-(2-aminoethyl)-3-aminopropyltrimethoxysilane; 3-methacryloxypropyltrimethoxysilane; N-[2-(vinylbenzylamino)ethyl]-3-aminopropyltrimethoxysilane; 3-glycidoxypropyltrimethoxysilane; vinyltriacetoxysilane; 3-chloropropyltrimethoxysilane; 2-(3,4-epoxycyclohexyl)-ethyltrimethoxysilane; 3-mercaptopropyltrimethoxysilane; and 2-mercaptoethyltrimethoxysilane.

Since thermoplastic materials are known to be generally non-reactive with the reactive organofunctional group of silane coupling agents resulting generally in non-bonding, there has been proposed certain non-cured compositions including a silane coupling agent and an alkoxymethyltriazine in order to provide effective bonds. Thus, in U.S. Patent 4,231,910 issued November 4, 1980 there is disclosed a primer composition which is said to consist essentially of 75-99 weight percent of an alkoxymethyltriazine which is a product of etherification of a methyloltriazine with a monohydric alcohol having four carbon atoms or less and 1-25 weight percent of an organosilicon compound selected from the group consisting of 3-glycidoxypropyltrimethoxysilane; 2-(3,4-epoxycyclohexyl)-ethyltrimethoxysilane, 3-mercaptopropyltrimethoxysilane, 2-mercaptoethyltrimethoxysilane and partial hydrolyzates thereof.

The primer composition of the '910 patent is said to be capable of improving adhesion between a thermoplastic and a solid substrate. The solid substrate is specified as being a siliceous material such is glass, quartz, ceramic, asbestos, silicone resins and glass fibers; metals such as aluminum, steel, copper, nickel, magnesium and titanium; metal oxides such as MgO, Fe₂O₃ and Al₂O₃; and organic solids such as wood, rubber and plastic materials. The primer may contain a solvent such as methanol, ethanol, isopropanol, butanol, xylene and mixtures thereof.

There is nothing in the '910 patent that would lead one to believe that such primer compositions could be useful or even operative where the solid substrate was living tissue.

Like hair, the fingernails and toenails are a specialized form of skin. Often termed "unguis" they are growths made up of the same material as the horns, claws, talons and hoofs of birds and animals. When moist, nails are soft and very plastic but upon dehydration become rigid and brittle. Nails grow outward from a reproductive matrix and lie along a nail bed which consists of modified skin within which are grooves containing blood vessels. The nails are formed of hard keratin designed to protect the fingers and toes from harm. Keratin is a type of protein high in sulfur content and is the most important constituent of these living human tissues. Other constituents include about fourteen percent water, calcium phosphate, carbonates and trace amounts of arsenic. Growth occurs at the rate of about 0.1 mm per day.

Surprisingly it has been discovered that the adhesion between a nail lacquer and the surface of a human fingernail or toenail can be enhanced by the interposition of a coating of the primer composition of the '910 patent. This extension of the teaching of the '910 patent to living human tissue is believed to be unexpected and the first application of that concept to the cosmetic field. In addition special solvents not taught in the '910 patent are an essential feature of the present invention.

Hence, the present invention relates to a method of adhering a coating of a nail lacquer to a nail surface, characterized in that a primer composition is applied to the nail surface prior to applying the nail lacquer coating, the primer composition consisting essentially of an alkoxymethyltriazine, an organosilicon compound selected from the group consisting of 3-glycidoxypropyltrimethyoxysilane; 2-(3, 4-epoxycyclohexyl)-ethyltrimethoxysilane, 3-mercaptopropyltrimethoxysilane,2-mercaptoethyltrimethoxysilane and partial hydrolyzates thereof and a solvent which is an ester selected from methyl acetate, ethyl acetate, butyl acetate, aryl acetate, butyl Cellosolve acetate, Cellosolve acetate and methyl Cellosolve acetate.

The present invention is believed to be an unexpected improvement over the technology of U.S. Patent No. 4,231,910. For example, nitrocellulose is not among one of the thermoplastic materials noted in the '910 patent. Further, the interaction between the silane coupling agent in the '910 patent is not the same interaction required for living human tissue as exemplified by fingernails and toenails. Unlike the -Si-O-Si- bond associated with the bonding of a silane coupling agent to a siliceous surface, bonding to living human tissue like a nail is effected by hydrogen bonding of ≡SiOH to the polar aminoacid keratin structure of the nail. The ≡SiOH structure is achieved by hydrolysis of ≡Si-OMe upon contact with the water in the nail. The oversimplified diagrams below illustrate these differences. 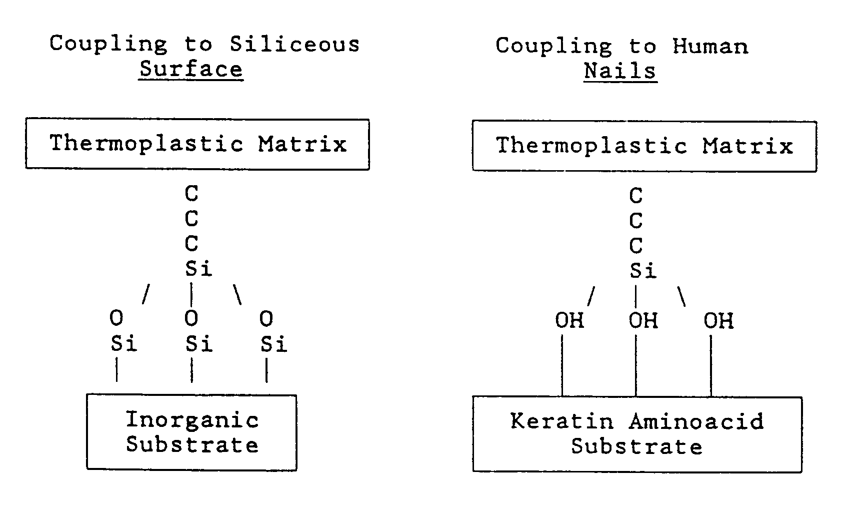
Because of the differences in the interactions, the stability of the bond between the silane coupling agent and the inorganic siliceous substrate will not be present in the case of the keratin aminoacid nail substrate. Accordingly, it would not be expected that the technology of the '910 patent would be applicable to living human tissue.

The invention is directed to a method of adhering a coating of a nail lacquer to a nail surface. The improvement resides in enhancing the adhesion between the nail lacquer coating and the nail surface by applying to the nail surface a primer composition prior to applying the nail lacquer coating. The primer composition consists essentially of an alkoxymethyltriazine, an organofunctional silane and a solvent.

These and other features, objects and advantages of the herein described present invention will become more apparent when considered in light of the following detailed description.

Nail lacquers and enamels with which the primer composition of the present invention is compatible may typically contain several ingredients among which are a primary film former, secondary film formers, plasticizers, solvents, colorants and fillers.

As the primary film former, nitrocellulose otherwise known as cellulose nitrate is the primary film former employed in the majority of nail polish formulations but there can also be employed materials such as thermoplastic cellulose acetate, thermoplastic cellulose acetate butyrate, thermoplastic ethyl cellulose, thermoplastic methacrylate polymers, vinyl polymers and sucrose acetate isobutyrate.

The plasticizer functions to control the flexibility and the elongation of the film. Plasticizers preferably should be nonvolatile, colorless, odorless and tasteless. Some examples of appropriate plasticizers which may be employed are dibutyl phthalate, tricresyl phosphate, dibutyl phthalate, dibutyl glycolate, dioctyl phthalate, camphor, castor oil, benzyl benzoate, tributyl phosphate, butyl acetal ricenoleate, glyceryl acetal ricenoleate, butyl stearate, tributoxy ethyl phosphate, triphenyl phosphate, triethyl citrate, tributyl citrate, tributyl acetyl citrate, dibutyl tartarate, dimethoxy ethyl phthalate and diamyl phthalate.

Solvents which may be used are esters such as methyl acetate, ethyl acetate, butyl acetate, amyl acetate, butyl Cellosolve acetate, Cellosolve acetate and methyl Cellosolve acetate. Ethyl acetate is the preferred solvent.

As a colorant there may be employed organic pigments such as The Cosmetics, Toiletries and Fragrance Association designated materials D and C Red Nos. 5-7, 10-13 and 34 and D and C Yellow Nos. 5 and 6. Cosmetic grade inorganic pigments may also be employed such as yellow and red iron oxides, brown iron oxide, iron blue, iron black, carbon black, purified titanium dioxide and bismuth oxychloride.

Secondary resins which may be included are dammar, elemi, shellac, sandarac, pontianak; and synthetic resins such as polyvinyl acetate, butyrates and sulfonamide resins. Fillers may be employed among which are nacreous pigments for the impartation of iridescence as titanium dioxide coated mica flakes, bismuth oxychloride coated mica flakes, colored aluminum chips and treated herring scales.

According to the present invention, the primer composition consists essentially of an alkoxymethyltriazine which is a product of etherification of a methyloltriazine with a monohydric alcohol having four carbon atoms or less, an organosilicon compound selected from the group consisting of 3-glycidoxypropyltrimethoxysilane; 2-(3,4-epoxycyclohexyl)-ethyltrimethoxysilane, 3-mercaptopropyltrimethoxysilane, silane, 2-mercaptoethyltrimethoxysilane and partial hydrolyzates thereof and an ester solvent.

Since this composition and methods of making the primer composition are described in detail in U.S. Patent No. 4,231,910, the primer will not be further described with any specificity other than to note that the preferred organosilicon compound is 3-glycidoxypropyltrimethoxysilane and that the solvent is an ester or more particularly one of ethyl acetate, butyl acetate, amyl acetate, butyl Cellosolve acetate, Cellosolve acetate or methyl Cellosolve acetate. Ethyl acetate is specially preferred.

The primer composition includes 0.1-10 weight percent of the silane preferably 0.1-1.0 percent, 1-10 weight percent of the alkoxymethyltriazine and 80-98.9 weight percent of the solvent. The primers are applied in one or more coatings with a brush applicator to the nails under ambient temperature. Evaporation of the solvent leaves a clear practically unobservable primer coating on the nail surface. A nitrocellulose nail lacquer is applied to the primer coated nail surface in the conventional fashion. Wear evaluations indicate extended wear in comparison to unprimed nails. In addition, nail coat compositions containing the primer of the present invention have been found to provide improved adhesion and wear resistance which are highly desirable and sought-after factors in the nail enamel market. The following examples illustrate the present invention and the concepts embodied therewithin.

### Example I

A primer composition was prepared by combining 0.5 weight percent of 3-glycidoxypropyltrimethoxysilane, 4.5 weight percent of CYMEL 303 which is a liquid grade of hexamethoxymethylmelamine and a trademark of American Cyanamid Company of Wayne New Jersey and 95 weight percent of ethyl acetate. CYMEL 301 and CYMEL 325 are similar American Cyanamid Company materials which may also be employed. This solution was applied with a brush applicator to the nails of several volunteers. One to four coatings of the primer was followed by one or more coatings of a nitrocellulose based colored nail lacquer after the primer coating had dried. Some of the nails of the volunteers were not primed. The volunteers reported that the primed nails exhibited extended wear in comparison to the nails which had not been primed.

## Claims

1. A method of adhering a coating of a nail lacquer to a nail surface, characterized in that a primer composition is applied to the nail surface prior to applying the nail lacquer coating, the primer composition consisting essentially of an alkoxymethyltriazine, an organosilicon compound selected from the group consisting of 3-glycidoxypropyltrimethyoxysilane; 2-(3,4-epoxycyclohexyl)-ethyltrimethoxysilane, 3-mercaptopropyltrimethoxysilane,2-mercaptoethyltrimethoxysilane and partial hydrolyzates thereof and a solvent which is an ester selected from methyl acetate, ethyl acetate, butyl acetate, aryl acetate, butyl Cellosolve acetate, Cellosolve acetate and methyl Cellosolve acetate.

2. A primer composition consisting essentially of an alkoxymethyltriazine, an organosilicon compound selected from the group consisting of 3-glycidoxypropryltrimethoxysilane; 2-(3,4-epoxycyclohexyl)-ethyltrimethoxysilane, 3-mercaptopropyltrimethoxysilane, 2-mercaptoethyltrimethoxysilane and partial hydrolysates thereof and a solvent which is an ester selected from the group consisting of ethyl acetate, butyl acetate, amyl acetate, butyl Cellosolve acetate, Cellosolve acetate and methyl Cellosolve acetate.

## Patentansprüche

1. Verfahren zum Haftendmachen einer Schicht aus einem Nagellack auf der Oberfläche eines Nagels, dadurch gekennzeichnet, daß man die Oberfläche des Nagels vor dem Aufbringen der Schicht aus dem Nagellack mit einer Grundierungsmischung behandelt, die im wesentlichen besteht aus einem Alkoxymethyltriazin, einer Organosiliciumverbindung, ausgewählt aus der Gruppe, bestehend aus 3-Glycidoxypropyltrimethoxysilan, 2-(3,4-Epoxycyclohexyl)-ethyltrimethoxysilan, 3-Mercaptopropyltrimethoxysilan, 2-Mercaptoethyltrimethoxysilan und Teilhydrolysaten dieser Stoffe, und einem Lösungsmittel, der ein Ester ist, ausgewählt aus Methylacetat, Ethylacetat, Butylacetat, Amylacetat, Butyl-Cellosolve-Acetat, Cellosolve-Acetat und Methyl-Cellosolve-Acetat.

2. Grundierungsmischung, im wesentlichen bestehend aus einem Alkoxymethyltriazin, einer Organosiliciumverbindung, ausgewählt aus der Gruppe, bestehend aus 3-Glycidoxypropyltrimethoxysilan, 2-(3,4-Epoxycyclohexyl)-ethyltrimethoxysilan, 3-Mercaptopropyltrimethoxysilan, 2-Mercaptoethyltrimethoxysilan und Teilhydrolysaten dieser Stoffe, und einem Lösungsmittel, der ein Ester ist, ausgewählt aus Ethylacetat, Butylacetat, Amylacetat, Butyl-Cellosolve-Acetat, Cellosolve-Acetat und Methyl-Cellosolve-Acetat.

## Revendications

1. Procédé pour faire adhérer une couche de vernis à ongles sur la surface d'un ongle, caractérisé en ce qu'on applique une composition primaire sur la surface de l'ongle avant d'appliquer la couche de vernis à ongle, la composition primaire étant essentiellement constituée d' une alcoxyméthyltriazine, d'un composé organosilicié choisi dans le groupe constitué du 3-glycidoxypropyltriméthoxysilane ; du 2-(3,4-époxycyclohexyl)éthyltriméthoxysilane, du 3-mercaptopropyltriméthoxysilane, du 2-mercaptoéthyltriméthoxysilane et des produits d'hydrolyse partielle de ceux-ci, et d'un solvant qui est un ester choisi parmi l'acétate de méthyle, l'acétate d'éthyle, l'acétate de butyle, l'acétate d'aryle, l'acétate de butyl Cellosolve, l'acétate de Cellosolve et l'acétate de méthyl Cellosolve.

2. Composition primaire essentiellement constituée d'une alcoxyméthyltriazine, d'un composé organosilicié choisi dans le groupe constitué du 3-glycidoxvpropyltriméthoxysilane ; du 2-(3,4-époxycyclohexyl)éthyltriméthoxysilane, du 3-mercaptopropyltriméthoxysilane, du 2-mercaptoéthyltriméthoxysilane et des produits d' hydrolyse partielle de ceux-ci, et d'un solvant qui est un ester choisi dans le groupe constitué de l'acétate d'éthyle, l'acétate de butyle, l'acétate d'amyle, l'acétate de butyl Cellosolve, l'acétate de Cellosolve et de l'acétate de méthyl Cellosolve.
